# EUROPEAN PATENT APPLICATION

(11) **EP 1 661 593 A1**
(43) Date of publication of application: **31.05.2006**
(21) Application number: 03817910.7
(22) Date of filing: 11.08.2003
(51) Int. Cl.: A61M 5/32

(54) **BUTTERFLY NEEDLE ASSEMBLY**

(71) Applicant: Yang, Chang-Ming, Miaoli County, Taiwan (CN)
(72) Inventor: Yang, Chang-Ming, Miaoli County, Taiwan (CN)
(74) Representative: Viering, Jentschura & Partner
(86) International application number: PCT/CN2003/000659
(87) International publication number: WO 2005/014086

(57) **Abstract**

A safety winged set includes essentially a winged needle and a winged needle cover, wherein the winged needle is provided with one lateral piece extending from two lateral sides thereof, respectively, the winged needle cover is provided with several gliding slots corresponding to the number and positions of the lateral pieces of the syringe, on the rear and upper end of one side gliding slot and on the rear and lower end of the other side gliding slot, an accommodating slot is provided, respectively, and a supporting portion is provided on the front end of the gliding slot. The bent portion of the lateral piece is bent upwardly, and then the holding portion of the winged needle cover is pressed by hand and pulled backwardly such that the injection needle is exposed in order to perform the injecting or drawing-out action. When the winged needle cover moves back to the rear end of the syringe, the supporting portion at the front end of the gliding slot clips to the lateral piece of the winged needle at the front end thereof such that the winged needle cover will not slid easily.

## Description

### BACKGROUND OF THE INVENTION

### 1. Field of the invention

The invention relates to a safety winged set, and in particular, to a safety winged set which is advantageous for preventing medical practitioner from risk during operation and which is easily operated.

### 2. DESCRIPTION OF THE PRIOR ART

Conventionally, a safety winged set is consisted of a winged needle and a winged needle cover. The winged needle is provided with several lateral pieces extended from sidewalls of a winged needle body, and an injection needle is provided in front of the winged needle body. The winged needle cover is in a cylindrical form having an accommodating aperture at its base. However, in the course of the blood collection by using the above-described conventional safety winged set, since the winged needle cover should be removed from the winged needle at first before injecting, after completion of the blood collection, the medical practitioner should return the winged needle cover, and just at this time, he (she) will be subject to be hurt by the injection needle if carelessly handled, and hence is susceptible to be infected.

Accordingly, the conventional art mentioned above has still many disadvantages and is not designed perfectly such that it needs to be improved immediately.

In view of the various disadvantages associated with the prior art safety winged set, the inventor of this application had devoted to improve it and, after studying intensively for many years, accomplished successfully the present safety winged set.

### SUMMARY OF THE INVENTION

It is an object of the present invention to provide a safety winged set characterized in that it can prevent from risk of being hurt for the medical practitioner in the course of blood collection.

It is another object of the present invention to provide a safety winged set characterized in that it is easily operated.

The safety winged set that can accomplishes the above-described objects according to the invention is consisted of:
a syringe, provided with one lateral piece extending from two lateral sides of the winged needle body, respectively, wherein each lateral piece is provided with a bent portion adjacent to the winged needle body, and the winged needle body is fitted with an injection needle at the front end thereof;
a winged needle cover, provided with several gliding slots on one or both sides thereof corresponding to the positions of the lateral pieces of the syringe, wherein on the upper end of one side gliding slot and the lower end of the other side gliding slot, an accommodating slot is provided, respectively, and the winged needle cover is provided with a holding portion on one side thereof.

### BRIEF DESCRIPTION OF THE DRAWINGS

The drawings disclose an illustrative embodiment of the present invention which serves to exemplify the various advantages and objects hereof, and are as follows:
Fig. 1 is a three-dimensional exploded view of the safety winged set according to the invention;
Fig. 1A is a partial enlarged view of the winged needle cover;
Fig. 2 is a three-dimensional out-looking view of the winged needle cover from another visual angle;
Fig. 3 is a schematic view of the safety winged set according to the invention in use;
Fig. 4 is a three-dimensional out-looking view of a first embodiment of the winged needle cover;
Fig. 5 is a three-dimensional exploded view of a second embodiment of the winged needle cover;
Fig. 6 is a three-dimensional exploded view of a first embodiment of the winged needle and the winged needle cover;
Fig. 7 is a three-dimensional exploded view of a second embodiment of the winged needle and the winged needle cover;
Fig. 8 is a three-dimensional exploded view of a third embodiment of the winged needle and the winged needle cover;
Fig. 9 is a three-dimensional exploded view of a fourth embodiment of the winged needle and the winged needle cover;
Fig. 9A is a three-dimensional exploded view of a fifth embodiment of the winged needle and the winged needle cover;
Fig. 9B is a three-dimensional exploded view of a sixth embodiment of the winged needle and the winged needle cover;
Fig. 9C is a three-dimensional exploded view of a seventh embodiment of the winged needle and the winged needle cover;
Fig. 10 is a three-dimensional exploded view of the safety winged set structure according to an embodiment of the invention;
Fig. 11 is a three-dimensional out-looking view of the test tube cap from another visual angle;
Fig. 11A is a three-dimensional cross-sectional view of the test tube cap;
Fig. 12 is a three-dimensional exploded view of an eighth embodiment of the winged needle and the winged needle cover;
Fig. 13 is a three-dimensional out-looking view of the embodiment of the lateral pieces;
Fig. 14 is a schematic view of a first embodiment of the inserting tube;
Fig. 15 is a schematic view of a second embodiment of the inserting tube;
Fig. 16 is a schematic view of a third embodiment of the inserting tube;

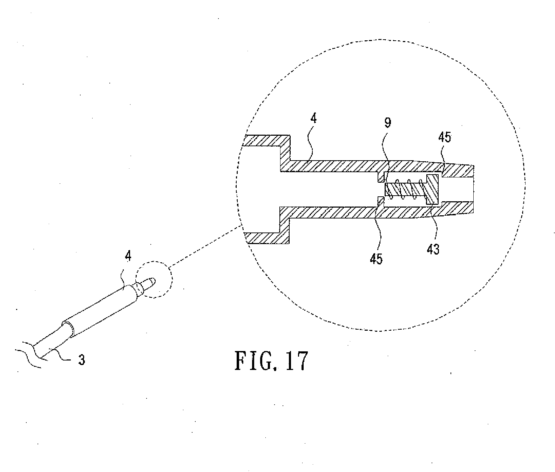

Fig. 17 is a schematic view of a fourth embodiment of the inserting tube;

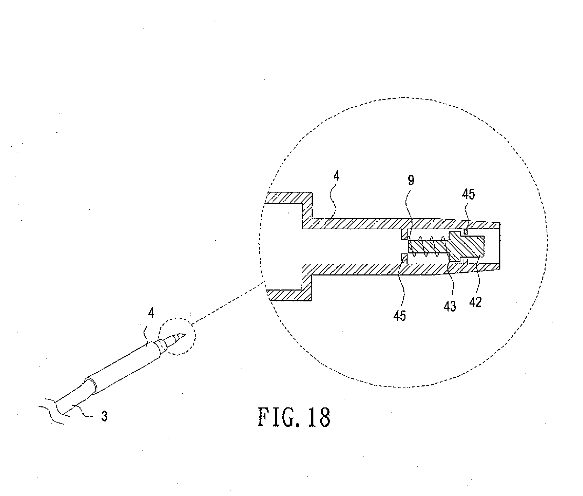

Fig. 18 is a schematic view of a fifth embodiment of the inserting tube;
Fig. 19 is a three-dimensional exploded view of a third embodiment of the winged needle cover;
Fig. 19A is a partial enlarged three-dimensional cross-sectional view of a third embodiment of the winged needle cover;
Fig. 19B is a three-dimensional out-looking view of a fifth embodiment of the winged needle cover; and
Fig. 19C is a three-dimensional out-looking view of a sixth embodiment of the winged needle cover.

### DETAILED DESCRIPTION OF THE PREFERRED EMBODIMENT

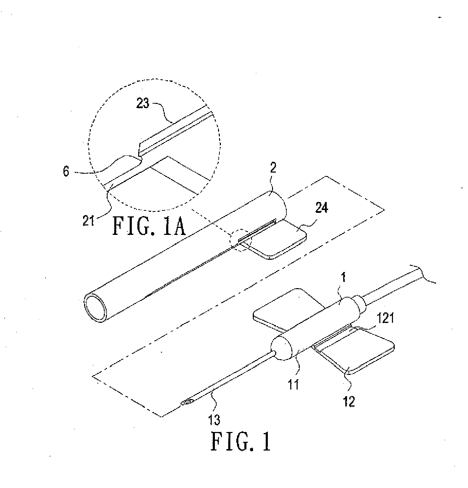

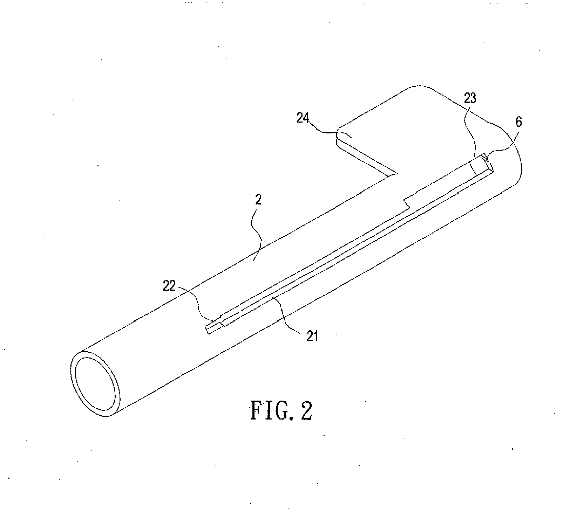

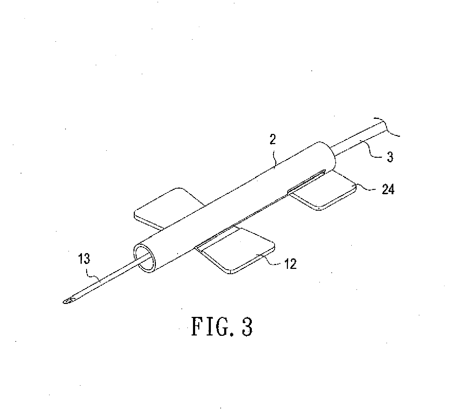

Referring to Figs. 1, 1A, 2 and 3, the safety winged set provided by the invention is consisted essentially of:
a winged needle 1, provided with one lateral piece 12 extending from two lateral sides of the winged needle body 11, respectively, wherein each lateral piece 12 is provide with a bent portion 121 adjacent to the winged needle body 11, the winged needle body 11 is fitted with an injection needle 13 at the front end thereof, and a flexible tube 3 is connected with the rear end of the winged needle 1;
a winged needle cover 2, provided with several gliding slots 21 on one or both sides thereof corresponding to the number and positions of the lateral pieces 12 of the winged needle 1, wherein on the front end of each gliding slot 21 is provided with a supporting portion 22, on the rear and upper end of one side gliding slot 21 and the rear and lower end of the other side gliding slot 21, an accommodating slot 23 is provided, respectively, a protruding part 6 is provided on the connecting portion of the accommodating slot 23 and the guiding slot 27, and the winged needle cover 2 is provided with a holding portion 24 on one side thereof.

Referring to Figs. 1, 1A, 2 and 3, in the safety winged set provided by the invention, the lateral piece 12 of the winged needle 1 is accommodated in the gliding slot 21 of the winged needle cover 2, and the winged needle cover 2 covers over the outside of the injection needle 13. Therefore, when the safety winged set provided by the invention is used, the bent portion 121 of the lateral piece 12 is bent upwardly, and then the holding portion 24 of the winged needle cover 2 is pressed by hand and pulled backwardly such that the injection needle 13 is exposed. When the winged needle cover 2 moves back to the rear end of the winged needle 1, the supporting portion 22 at the front end of the gliding slot 21 clips to the lateral piece 12 of the winged needle 1 at the front end thereof such that the winged needle cover 2 will not slid easily. Then, the injection needle 13 is used to puncture into a subject. After the safety winged set provided by the invention completes its action, the injection needle 13 is firstly drawn out of the subject, and then the holding portion 24 of the winged needle cover 2 is pressed by hand and pushed forwardly such that the winged needle cover 2 covers over the outside of the injection needle 13. Then, the winged needle cover 2 is rotated in a manner that the lateral piece 12 of the winged needle 1 is accommodated in the accommodating slot 23 of the winged needle cover 2, and the lateral piece 12 is fastened to the accommodating slot 23 by means of the stopping function of the protruding part 6. As described above, since the safety winged set provided by the invention use the accommodating slot 23 on the gliding slot 21 of the winged needle cover 2 to accommodate the lateral piece 12 of the winged needle 1 and the protruding part 6 is employed to stop the lateral piece 12 such that the winged needle cover 2 is fixed at the front end of the winged needle 1, the medical practitioner shall never be punctured accidentally by the injection needle 13 so as to prevent from risk of being infected.

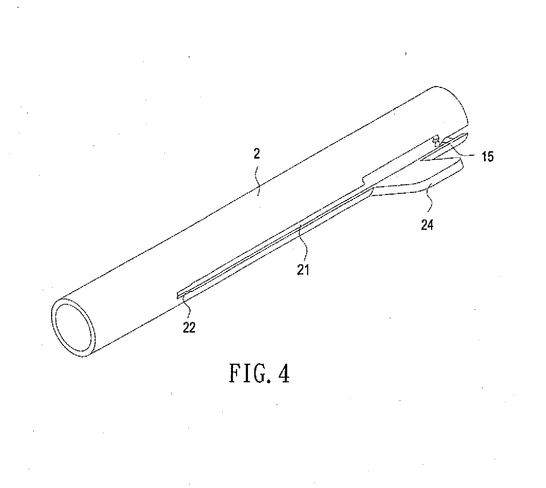

Referring to Fig. 4, in the safety winged set provided by the present invention, the gliding slot 21 of the winged needle cover 2 penetrates through the rear end of the winged needle cover 2 such that a conventional winged needle 1 can be used. In addition, the gliding slot 21 is provided with a stopping block 15 at a position adjacent to the rear end thereof. The front wall surface of the stopping block 15 is perpendicular to the gliding slot 21. The rear wall surface of the stopping block 15 is an incline. Therefore, the lateral piece 12 of the winged needle 1 for a conventional safety winged set can penetrate into the gliding slot 21 by means of the rear inclined plane of the stopping block 15. When the lateral piece 12 of the winged needle 1 penetrates into the gliding slot 21, the vertical surface at the front end of the stopping block 15 can prevent the lateral piece 12 from being drawn out from the rear end of the gliding slot 21.

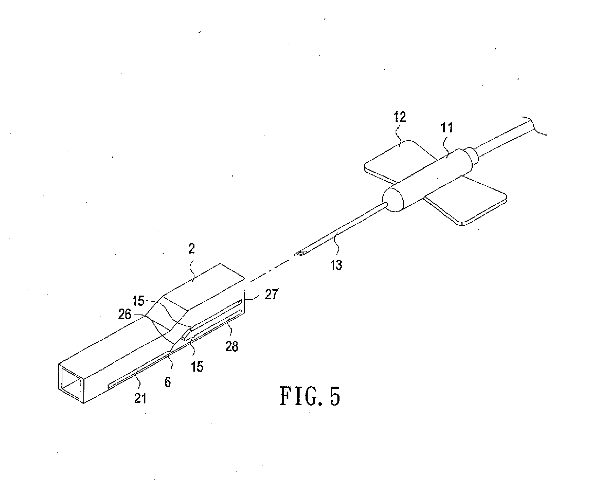

Referring to Fig. 5, in the safety winged set provided by the present invention, on the upper end of the gliding slot 21 of the winged needle cover 2, there is a guiding track 26 connected to a guiding slot 27, and a protruding part 6 is provided on the connecting portion of the guiding track 26 and the gliding slot 21. On the rear end of the gliding slot 21, a stopping block 15 is provided to partition an accommodating portion 28 at the rear end of the gliding slot 21. Before the safety winged set provided by the invention is used, the lateral piece 12 of the winged needle 1 is accommodated in the guiding slot 27 of the winged needle cover 2. Whereas, when the safety winged set provided by the invention is used, the winged needle cover 2 should be pulled backwardly such that the lateral piece 12 of the winged needle 1 will glide to the front end of the gliding slot 21, and the lateral piece 12 is fastened to the front end of the winged needle cover 2 by means of the protruding part 6 at the connecting portion of the guiding track 26 and the gliding slot 21. After the safety winged set provided by the invention completes its action, the winged needle cover 2 is pushed forwardly by exerting a force such that the lateral piece 12 penetrates the stopping block 15 at the rear end of the gliding slot 21 into the accommodating portion 28. In addition, the rear wall surface of the stopping block 15 is perpendicular to the gliding slot 21. Therefore, the lateral piece 12 can be fastened to the rear side of the winged needle cover 2.

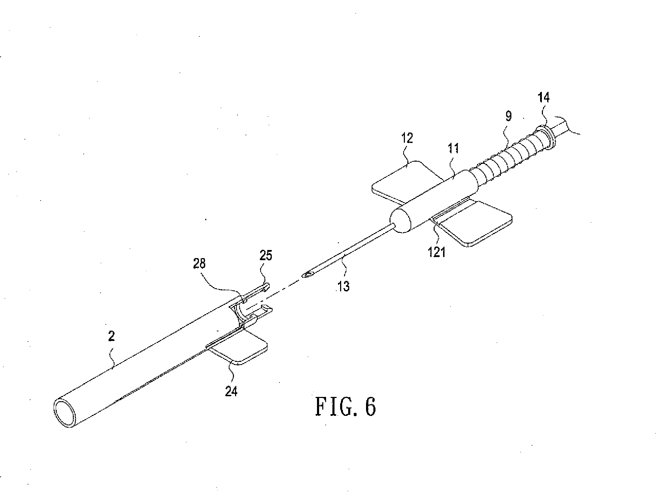

Referring to Fig. 6, in the safety winged set provided by the present invention, a spring 9 is sleeved on the rear portion of the winged needle 1, and a ring portion 14 is fixed against the rear end of the spring 9. Furthermore, a hook 25 extending from the rear portion of the winged needle cover 2 can be engaged with the rear wall surface of the ring portion 14. When the winged needle cover 2 is pulled to the rear end of the winged needle 1, the spring 9 is pushed backwardly by means of the holding portion 24, and the hook 25 is engaged with the rear wall surface of the ring portion 14 such that the winged needle cover 2 is fixed to the rear end of the winged needle 1. After the safety winged set provided by the invention completes its action, the hook 25 is pulled open, and the winged needle cover 2 will push back to the front end of the winged needle 1 by means of the elastic force provided by the spring 9. Alternatively, the winged needle cover 2 can be also provided with a protruding block 28 at the interior edge of the rear end thereof. When the winged needle cover 2 is pulled to the rear end of the winged needle 1, the protruding block 28 can be used to push the spring backwardly.

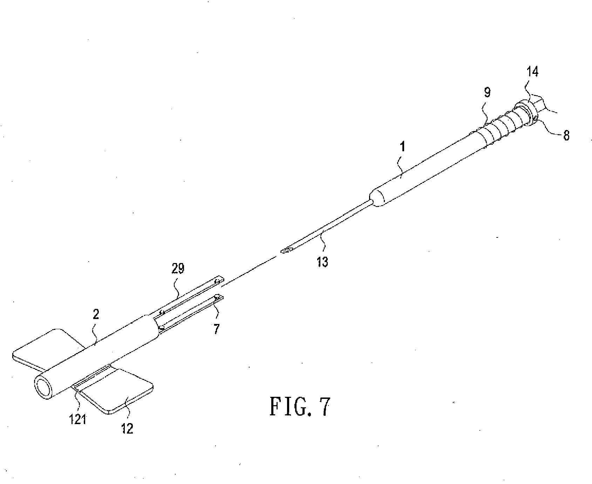

Referring to Fig. 7, in the safety winged set provided by the present invention, a spring 9 is sleeved on the rear portion of the winged needle 1, and a ring portion 14 is fixed against the rear end of the spring 9. In addition, several fastening holes 8 are provided on the ring portion 14, the lateral pieces 12 of the winged needle 1 are provided on the lateral sides of the winged needle cover 2, and several resilience pieces 29 extending from the rear end of the winged needle cover 2 have protruding fasteners 7 on the interior edges at the front and the rear ends thereof, respectively. Before the safety winged set provided by the invention is used, the protruding fasteners 7 at the ends of the resilience pieces 29 are engaged in the corresponding fastening holes 8. Thus, when the safety winged set provided by the invention is used, the resilience pieces should be firstly pulled open such that the protruding fasteners 7 will detach from the fastening holes 8 of the ring portion 14. Then, the winged needle cover 2 is pulled backwardly, and the protruding fasteners 7 at the front ends of the resilience pieces 29 are engaged in the fastening holes 8 of the ring portion 14 such that the winged needle cover 2 is fixed to the rear end of the winged needle 1. Alternatively, the fastening holes 8 on the ring portion 14 can be replaced by the protruding fasteners 7; whereas, the protruding fasteners 7 on the resilience pieces 29 can be replaced by the fastening holes 8. Alternatively, no fastening hole 8 is provided on the ring portion 14, and the protruding fastener 7 provided on the resilience piece 29 of the winged needle cover 2 can be replaced by the hook 25.

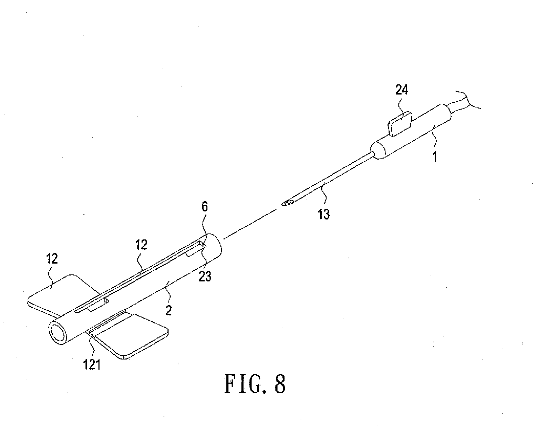

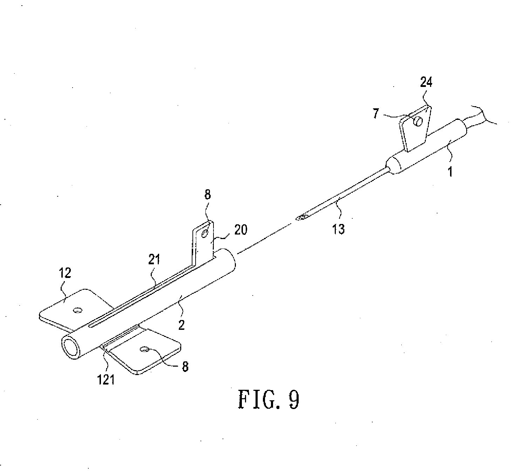

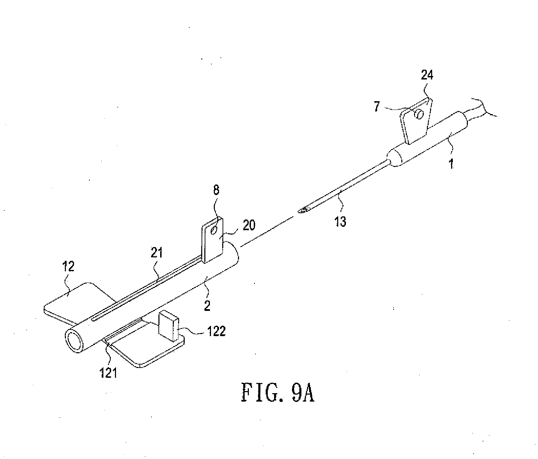

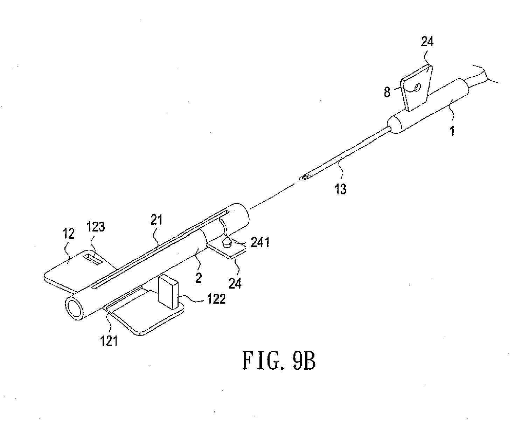

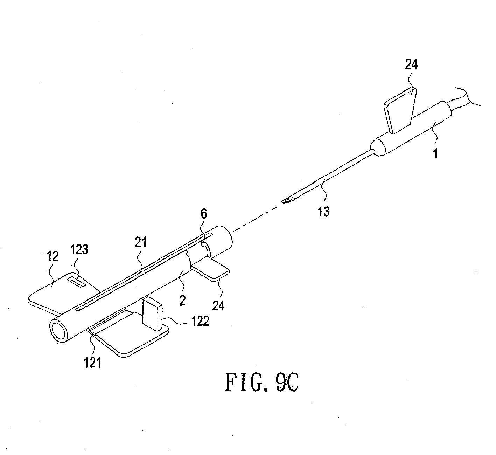

Referring to Figs. 8 and 9, in the safety winged set provided by the present invention, the holding portion 24 of the winged needle cover 2 can be provided on the outer periphery of the winged needle 1, the lateral pieces 12 of the winged needle 1 are provided on the lateral sides of the winged needle cover 2, and the winged needle cover 2 is provided thereon with the gliding slot 21 for the holding portion 24 of the winged needle 1 to penetrate therethrough. At the front and the rear ends of the gliding slot 21, an accommodating slot 23 is provided, respectively. A protruding part 6 is provided on the connecting portion of the accommodating slot 23 and the gliding slot 21. In such way, when the winged needle cover 2 glides between the front and the rear ends of the winged needle 1, the accommodating slot 23 at the front and the rear ends thereof will accommodate the holding portion 24 of the winged needle 1 such that the winged needle cover 2 is fixed to the front and the rear ends of the winged needle I. Alternatively, the rear portions of the winged needle 1 and the winged needle cover 2 can also provided with springs 9, ring portions 14 and the hooks 25 or the resilience pieces 29 as described in Figs. 6 and 7. Alternative, no accommodating slot 23 is provided on the front and the rear ends of the winged needle cover 2, the lateral pieces 12 of the winged needle cover 2 are provided with the fastening hole 8, the holding portion 24 of the winged needle 1 at its side edge is provided with the protruding fastener 7 to be engaged with the fastening hole 8, one or more fastening pieces 20 are provided on the rear end of the gliding slot 21, and the fastening piece 20 is provided with the fastening hole 8 to be engaged with the protruding fastener 7 of the holding portion 24.

Referring to Figs. 9A, 9B and 9C, in the safety winged set provided by the present invention, the front and the rear ends of the gliding slot 21 of the winged needle cover 2 can provided with no accommodating slot 23. At the rear end of the lateral piece 12 on one side of the winged needle cover 2, a stopping piece 122 extends upwardly therefrom. At the rear end of the gliding slot 21, one or more fastening pieces 20 are provided. The fastening piece 20 has thereon a fastening hole 8. The holding portion 24 of the winged needle 1 is provided with a protruding fastener 7 at the position corresponding to the fastening hole 8 of the fastening piece 20. When the winged needle cover 2 glides to the rear end of the winged needle 1 and the injection needle 13 penetrates out the winged needle cover 2, by pulling the lateral piece 12 upwardly, the stopping piece 122 on one side at the rear end thereof will stop the rear end of the holding portion 24 so as to prevent the winged needle 1 from gliding backwardly when the winged needle 1 is punctured into a medical practitioner. When the action of injection or withdrawal is completed, the winged needle cover 2 is pushed forwardly to cover the injection needle 13, and the protruding fastener 7 on the holding portion is engaged with the fastening hole 8 of the fastening piece 20 such that the winged needle cover 2 is fixed to the front end of the winged needle 1. Furthermore, the lateral piece 12 of the winged needle cover 2, without the stopping piece 122, can be provided with a fastening aperture 123. When the lateral piece 12 is pulled upwardly, the stopping piece 122 can be directly engaged into the fastening aperture 123 so as to stop the stopping piece 122 behind the holding portion 24. Alternatively, an aperture is provided on the lateral edge of the gliding slot 21 and extends to the lateral edge of the winged needle cover 2, and a fastening piece 20 is extended from the aperture. A stopping fastener 241 is provided on the fastening piece 20, and the protruding fastener 7 on the holding portion 24 of the winged needle 1 is replaced by the fastening hole 8. Alternatively, no fastening hole 8 is provided on the holding portion 24 of the winged needle 1, and no fastening piece 20 is provided on the winged needle cover 2. On the upper or lower end of the gliding slot 21 adjacent to the rear end thereof, an accommodating slot 23 is provided, and a protruding part 6 is provided on the connecting portion of the accommodating slot 23 and the guiding slot 27. When the winged needle cover 2 glides to the front end of the winged needle 1, by rotating the winged needle cover 2, the holding portion 24 of the winged needle 1 is accommodated in the accommodating slot 23 of the winged needle cover 2, and the holding portion 24 is fixed in the accommodating slot 23 by means of the protruding part 6.

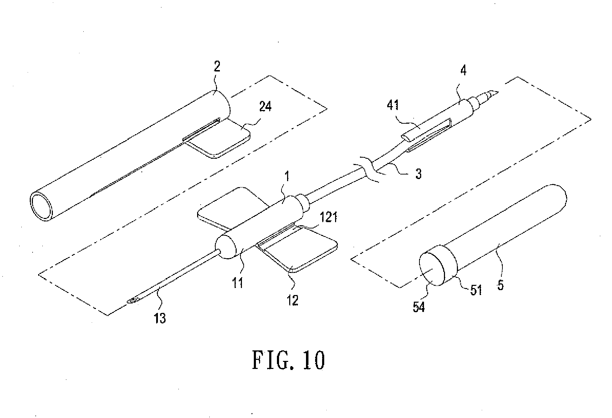

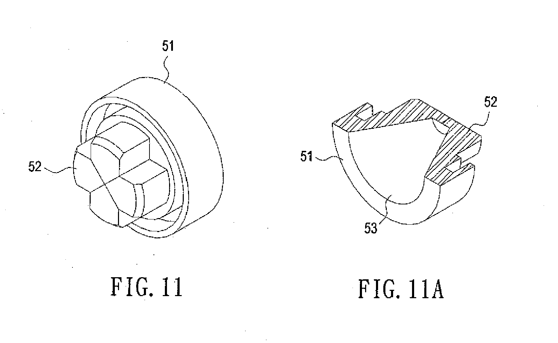

Referring to Figs. 10, 11 and 11 A, in the safety winged set provided by the present invention, the rear end of the winged needle 1 is connected to an inserting tube 4 via the flexible tube 3. The inserting tube 4 can be inserted into the test tube 5. A clamp portion 41 is provided on the rear end of the inserting tube 4. A test tube cap 51 is sleeved on the opening end of the test tube 5. Several claw-shaped clamping heads 52 are provided on the rear end of the test tube cap 51. The claw-shaped clamping heads 52 compress with each other to form seal. A guiding slot 53 is provided on the front end of the test tube cap 51 and connected with the rear ends of the claw-shaped clamping heads 52. The wall surface of the guiding slot 53 is an incline tapered inwardly. Alternatively, a membrane 54 is provided at the outer periphery of guiding slot 53 of the test tube cap 51. When the safety winged set according to the invention is used to collect blood, the injection needle 13 at the front end of the winged needle 1 is punctured into the subject, the inserting tube 4 behind the flexible tube 3 pierces through the membrane 54 on the test tube cap 51, and the inserting tube 4 pushes into the claw-shaped clamping heads 52 at the rear end of the test tube cap 51 via the guidance of the incline of the guiding slot 53. Then, by means of the attraction due to the negative pressure generated within the test tube 5, the blood of the subject is draw out to the test tube 5 by the injection needle 13 via the flexible tube 3. Meanwhile, if the blood of the subject is to be drawn out, only the rear end of the clamp portion 41 is to be pressed. Accordingly, the clamp portion 41 will compress the flexible tube 3 such that the blood no longer passes through the flexible tube 3. Then, the inserting tube 4 is inserted into another test tube 5, and the clamp portion 41 is loosened to allow the blood to pass through. In addition, since the inserting tube 4 is provided with the clamp portion 41 at the rear end thereof, when a new test tube 5 is replaced, the user can directly compress the clamp portion 41 to prevent the blood from passing through the flexible tube 3. In such way, when a new test tube 5 is replaced, the blood will no longer flow out the inserting tube 4 so as to prevent the medical practitioner from being infected. Alternatively, the membrane 54 provided at the outer periphery of guiding slot 53 can also be replaced by a cap. Also, the rear end of the winged needle 1 can be connected to an intravenous drip tube 30 via the flexible tube 3 in order to keep connection with the intravenous drip.

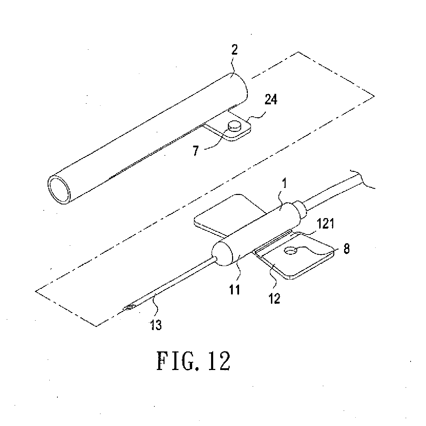

Referring to Fig. 12, in the safety winged set provided by the present invention, no accommodating slot 23 is provided on the upper end of the gliding slot 21 of the winged needle cover 2. Whereas, a protruding fastener 7 is provided on the holding portion 24, and the lateral piece 12 of the winged needle 1 is provided with a fastening hole 8 at the position corresponding to the protruding fastener 7. Therefore, when the safety winged set provided by the invention is used, the lateral piece 12 of the winged needle 1 should be firstly pulled upwardly by means of the bent portion 121 such that the fastening hole 8 on the lateral piece 12 is detached from the protruding fastener 7 on the holding portion 24. Then, the holding portion 24 is pressed by hand, and the winged needle cover 2 is pulled backwardly. After the safety winged set provided by the invention completes its blood collecting action, the holding portion 24 is pressed by hand, and the winged needle cover 2 is pushed forwardly. Then, the protruding fastener 7 on the holding portion 24 is engaged into the fastening hole 8 on the lateral piece 12, such that the winged needle cover 2 is fixed onto the front end of the winged needle 1 and the injection needle 13 is covered. Alternatively, the protruding fastener 7 on the holding portion 24 can be replaced by the fastening hole 8, and the fastening hole 8 on the lateral piece 12 can be replaced by the protruding fastener 7.

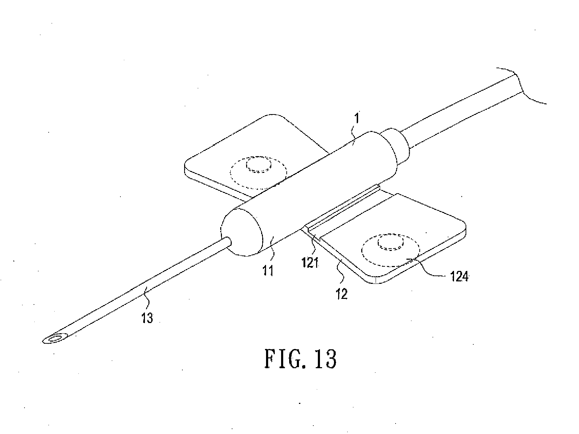

Referring to Fig. 13, in the safety winged set provided by the present invention, the lateral piece 12 of the winged needle 1 is provided with one or more suckers 124 at the lower portion thereof. Therefore, when the safety winged set provided by the invention is used to inject or withdraw blood, the suckers 124 at the lower portion of the lateral piece 12 are adhered to the skin of the subject. In addition, the sucker 124 can also be replaced by viscose or other adhesive materials having viscous properties. Alternatively, as shown in Fig. 9, the holding portion 24 of the winged needle 1 or the fastening piece 20 of the winged needle cover 2 is provided a sucker 124. Also, the sucker can be provided on the rear and lower portion of the syringe.

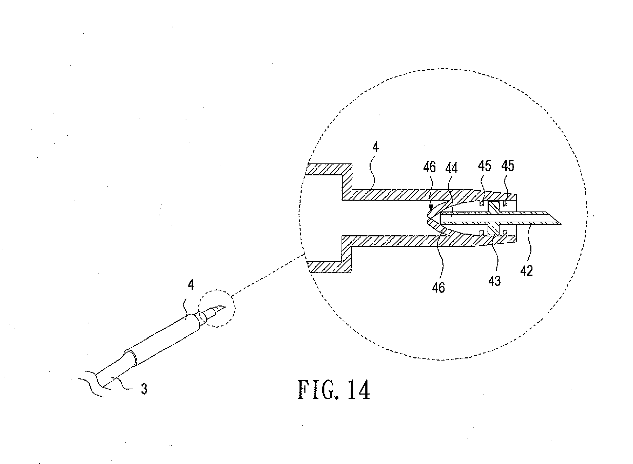

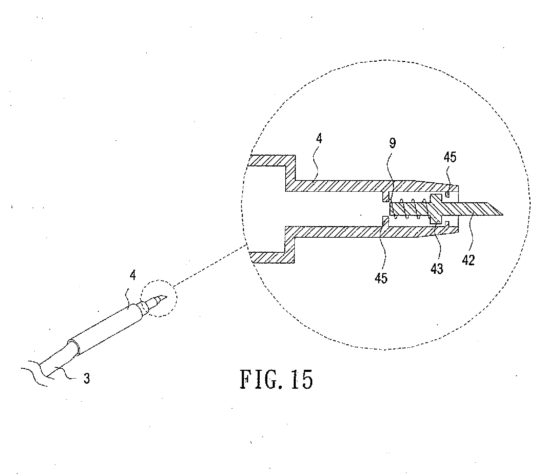

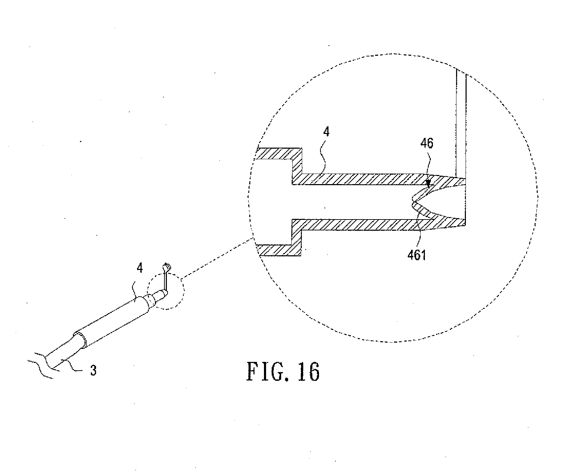

Referring to Figs. 10, 14, 15 and 16, no clamp portion 41 is provided at the rear end of the inserting tube 4, and an interposing element 42 is provided within the inserting tube 4. A holding element 43 is provided around the periphery of the interposing element 42, and a flow-directing hole 44 penetrates through the interposing element 42. A ring protruding stopper 45 is provided on the interior wall surface of the inserting tube 4 at the front and rear ends of the holding element 43. A non-return structure 46 constructed of several blades 461 is provided behind the ring protruding stopper 45 at the rear end of the inserting tube 4. When the safety winged set provided by the invention is used to collect blood, the interposing element 42 of the inserting tube 4 is used to pierce through the membrane 54 on the test tube cap 51, and the inserting tube 4 pushes into the claw-shaped clamping heads 52 at the rear end of the test tube cap 51 via the guidance of the incline of the guiding slot 53. At that time when the interposing element 42 pushes into the claw-shaped clamping heads 52, the blades 461 of the non-return structure 46 are pushed open due to the reacting force, such that the blood flow into the test tube 5 via the backflow hole. Alternatively, no flow-directing hole 44 is provided on the interposing element 42, the holding element 43 can be provided on the rear end or the middle outer periphery of the interposing element 42, and the ring protruding stopper 45 at the front end of the holding element 43 of the inserting tube 4 extends to the front end of the inserting tube 4, such that by inserting the test tube 5 or other tube into the inserting tube, the holding element 43 pushes into the inserting tube 4. At that time, the blood flows into the test tube 5 via the space between holding element 43 and the front and the rear ring protruding stoppers 45. Alternatively, no non-return structure 46 is provided within the inserting tube 4, and a spring 9 is sleeved around the outer periphery of the inserting tube 4 at the rear end thereof. The front end of the spring 9 is connected to the holding element 43 of the interposing element 42 at the rear end. The rear end of the spring 9 is fixed against the front end of the ring protruding stopper 45 at the rear end of the inserting tube 4. By means of the elastic force produced by the spring 9, the holding element 43 of the interposing element 42 is pushed against the rear wall surface of the ring protruding stopper 45 at the front end of the inserting tube 4. In addition, no flow-directing hole 44 is provided on the interposing element 42. When the safety winged set provided by the invention is used to collect blood, the interposing element 42 is drawn back in response to the reacting force generated from pulling open of the claw-shaped clamping heads 52. At that time, the blood flows into the test tube 5 via the space between holding element 43 and the front and the rear ring protruding stoppers 45. Alternatively, no interposing element 42 is provided within the inserting tube 4, no ring protruding stopper 45 is provided on the interior wall of the inserting tube 4, and the non-return structure 46 is directly provided on the interior wall of the inserting tube 4 at the front end.

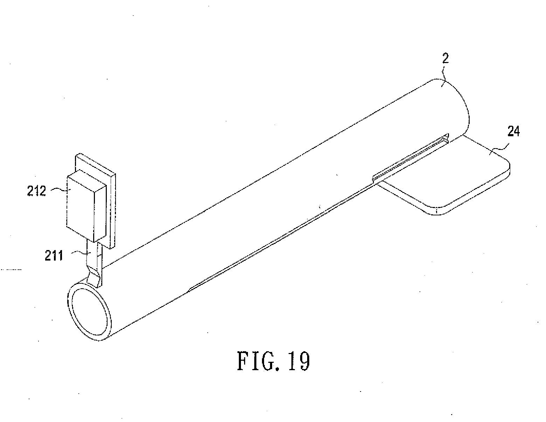

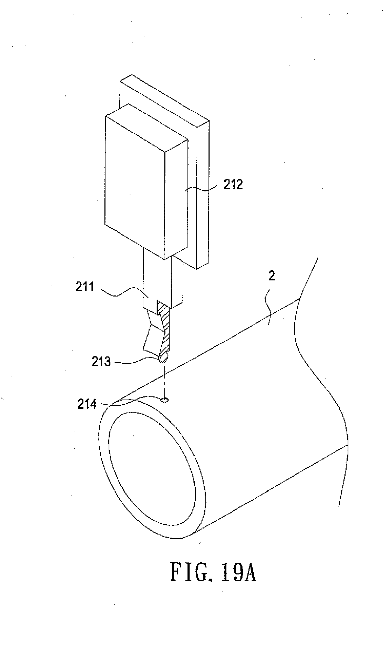

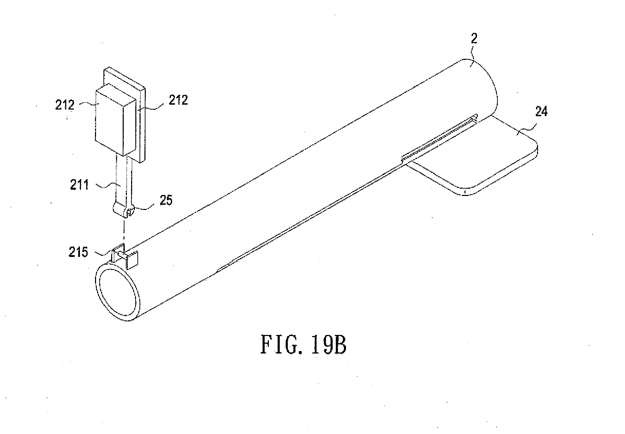

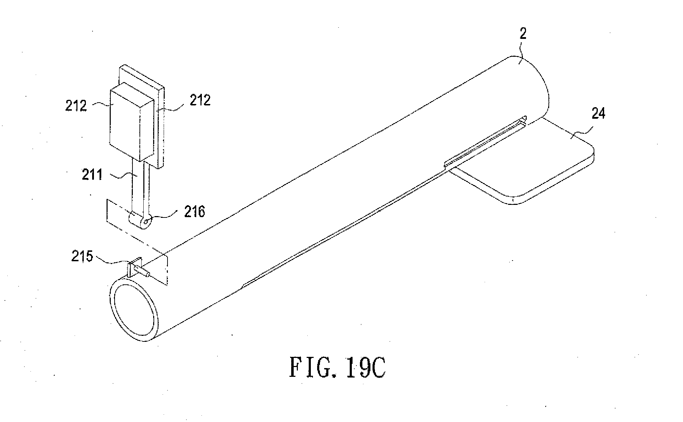

Referring to Figs. 19, 19A, 19B and 19C, in the safety winged set provided by the present invention, the outer periphery at the front end of the winged needle cover 2 is attached thereto a bent piece 211, and a hemostatic cotton 212 is connected to the upper end of the bent piece 211. After the action of injection or withdrawal by the safety winged set provided by the invention is completed, the bent piece 211 is bent toward the front end of the winged needle cover 2 in order to cover the hemostatic cotton 212 onto the injecting site of the subject by the injection needle 13. Then, the winged needle 1 is pulled backwardly such that the injection needle 13 is drawn out from the injecting site of the subject. Meanwhile, since the hemostatic cotton 212 is compressed and covered on the injecting site, the object of hemostasis is achieved. Alternatively, an inserting bar 213 extends from the lower portion of the bent piece 211, and an inserting hole 214 to be inserted by the inserting bar 213 is provided on the winged needle cover 2 such that the bent piece 211 is fixed onto the winged needle cover 2. Alternatively, the inserting bar 213 at the lower portion of the bent piece 211 can be replaced by the hook 25, and a shaft bar 215 to be engaged with the hook 25 is provided on the winged needle cover 2. Alternatively, a shaft hole 216 is provided at the lower portion of the bent piece 211 to be sleeved on the shaft bar 215 such that the bent piece 211 is fixed onto the winged needle cover 2.

Many changes and modifications in the above described embodiment of the invention can, of course, be carried out without departing from the scope thereof. Accordingly, to promote the progress in science and the useful arts, the invention is disclosed and is intended to be limited only by the scope of the appended claims.

### [represent symbols of major parts]

| | | | |
|---|---|---|---|
| 1 | winged needle | 41 | clamp portion |
| 2 | winged needle cover | 42 | interposing element |
| 3 | flexible tube | 43 | holding element |
| 4 | inserting tube | 44 | flow-directing hole |
| 5 | test tube | 45 | ring protruding stopper |
| 6 | protruding part | 46 | non-return structure |
| 7 | protruding fastener | 51 | test tube cap |
| 8 | fastening hole | 52 | claw-shaped clamping head |
| 11 | winged needle body | 53 | guiding slot |
| 12 | lateral pieces | 54 | membrane |
| 13 | injection needle | 121 | bent portion |
| 14 | ring portion | 122 | stopping piece |
| 15 | stopping block | 123 | fastening aperture |
| 20 | fastening piece | 124 | sucker |
| 21 | gliding slot | 211 | bent piece |
| 22 | supporting portion | 212 | hemostatic cotton |
| 23 | accommodating slot | 213 | inserting bar |
| 24 | holding portion | 214 | inserting hole |
| 25 | hook | 215 | shaft bar |
| 26 | guiding track | 241 | stopping fastener |
| 27 | guiding slot | 216 | shaft hole |
| 28 | accommodating portion | 461 | blade |
| 29 | resilience piece | | |

## Claims

1. A safety winged set comprising:
a winged needle, provided with one lateral piece extending from two lateral sides of the winged needle body, respectively, wherein said winged needle body is fitted with an injection needle at the front end thereof, and a flexible tube is connected with the rear end of said winged needle ;
a winged needle cover, provided with several gliding slots on one or both sides thereof corresponding to the positions of said lateral pieces of said syringe, wherein on the upper end of one side gliding slot and the lower end of the other side gliding slot, an accommodating slot is provided, respectively, and said winged needle cover is provided with a holding portion on one side thereof.

2. The safety winged set according to claim 1, wherein said lateral piece is provided on one lateral side of said winged needle body.

3. The safety winged set according to claim 1, wherein each lateral piece is provided with a bent portion adjacent to said winged needle body.

4. The safety winged set according to claim 1, wherein a protruding part is provided on the connecting portion of said accommodating slot and said gliding slot.

5. The safety winged set according to claim 1, wherein no accommodating slot is provided on the upper end of said winged needle cover, said holding portion is provided with a protruding fastener, and said lateral piece of said winged needle is provided with a fastening hole at a position corresponding to said protruding fastener.

6. The safety winged set according to claim 5, wherein said fastening holes on said holding portion can be replaced by said protruding fastener, whereas said protruding fastener on said lateral piece can be replaced by said fastening hole.

7. The safety winged set according to claim 1, wherein a spring is sleeved on the rear portion of said syringe, a ring portion is fixed against the rear end of said spring, a hook extending from the rear end of said winged needle cover is engaged with the rear wall surface of said ring portion, and said winged needle cover is provided with a protruding block at the interior edge of the rear end thereof.

8. The safety winged set according to claim 1, wherein said winged needle cover is provided with a protruding block at the interior edge of the rear end thereof.

9. The safety winged set according to claim 1, wherein a spring is sleeved on the rear portion of said syringe, a ring portion is fixed against the rear end of said spring, several fastening holes are provided on said ring portion, said lateral piece of said winged needle is provided on said lateral side of said winged needle cover, several resilience pieces extending from the rear end of said winged needle cover have protruding fasteners on the interior edges at the front and the rear ends thereof, respectively.

10. The safety winged set according to claim 9, wherein several protruding fastener are provided on said ring portion, and each resilience piece is provided with a fastening hole on the rear end thereof.

11. The safety winged set according to claim 9, wherein no fastening hole is provided on said ring portion, and said protruding fastener on the front and rear ends of said resilience piece is replaced by a hook.

12. The safety winged set according to claim 1, wherein said holding portion of said winged needle cover is provided on the outer periphery of said syringel, said lateral piece of said winged needle is provided on the lateral side of said winged needle cover, said winged needle cover is provided thereon with said gliding slot for said holding portion of said winged needle to penetrate therethrough, at the front and the rear ends of said gliding slot, an accommodating slot is provided, respectively, and a protruding part is provided on the connecting portion of said accommodating slot and said gliding slot.

13. The safety winged set according to claim 12, wherein no accommodating slot is provided on the front and the rear ends of said gliding slot of said winged needle cover, said lateral piece of said winged needle cover is provided with said fastening hole, said holding portion of said winged needle at its side edge is provided with said protruding fastener to be engaged with said fastening hole, one or more fastening pieces are provided on the rear end of said gliding slot, and said fastening piece is provided with said fastening hole to be engaged with the protruding fastener of said holding portion.

14. The safety winged set according to claim 13, wherein no accommodating slot is provided on said lateral piece of said winged needle cover, at the rear end of said lateral piece on one side of said winged needle cover, a stopping piece extends upwardly therefrom, and only one side of said holding portion is provided with a protruding fastener at the position corresponding to said fastening hole of said fastening piece.

15. The safety winged set according to claim 14, wherein said lateral piece of said winged needle cover without said stopping piece is provided with a fastening aperture.

16. The safety winged set according to claim 13, wherein an aperture is provided on the lateral edge of said gliding slot and extends to the lateral edge of said winged needle cover, a fastening piece is extended from said aperture, a stopping fastener is provided on said fastening piece, and said protruding fastener on said holding portion of the winged needle is replaced by said fastening hole.

17. The safety winged set according to claim 16, wherein no fastening hole is provided on said holding portion of said syringe, no fastening piece and no aperture is provided on said winged needle cover, on the upper or lower end of said gliding slot adjacent to the rear end thereof, an accommodating slot is provided, and a protruding part is provided on the connecting portion of said accommodating slot and said guiding slot.

18. The safety winged set according to claim 1, wherein said gliding slot of said winged needle cover penetrates through the rear end of said winged needle cover, said gliding slot is provided with a stopping block at a position adjacent to the rear end thereof, the front wall surface of said stopping block is perpendicular to said gliding slot, and the rear wall surface of said stopping block is an incline.

19. The safety winged set according to claim 1, wherein on the upper end of said gliding slot of said winged needle cover, there is a guiding track connected to a guiding slot, a protruding part is provided on the connecting portion of said guiding track and said gliding slot, and on the rear end of the gliding slot, a stopping block is provided to partition an accommodating portion at the rear end of said gliding slot.

20. The safety winged set according to claim 1, wherein the rear end of said winged needle is connected to an inserting tube to be inserted into said test tube via said flexible tube, and a clamp portion is provided on the rear end of said inserting tube.

21. The safety winged set according to claim 20, wherein a test tube cap is sleeved on the opening end of said test tube, several claw-shaped clamping heads are provided on the rear end of said test tube cap, said claw-shaped clamping heads compress with each other to form seal, a guiding slot is provided on the front end of said test tube cap and connected with the rear ends of said claw-shaped clamping heads, and the wall surface of said guiding slot is an incline tapered inwardly.

22. The safety winged set according to claim 21, wherein the outer periphery of said guiding slot is provided with a membrane.

23. The safety winged set according to claim 22, wherein said membrane at the outer periphery of said guiding slot is replaced by a cap.

24. The safety winged set according to claim 1, wherein said lateral piece of said winged needle is provided with one or more suckers at the lower portion thereof, and said sucker is replaced by viscose or other adhesive materials having viscous properties.

25. The safety winged set according to claim 1, wherein said holding portion of said winged needle or said fastening piece of said winged needle cover is provided a sucker, and said sucker is provided on the rear and lower portion of said syringe.

26. The safety winged set according to claim 20, wherein no clamp portion is provided at the rear end of said inserting tube, an interposing element is provided within said inserting tube, a holding element is provided around the periphery of said interposing element, said interposing element is provided with a flow-directing hole penetrating therethrough, a ring protruding stopper is provided on the interior wall surface of said inserting tube at the front and rear ends of said holding element, and a non-return structure constructed of several blades is provided behind said ring protruding stopper at the rear end of said inserting tube.

27. The safety winged set according to claim 20, wherein no non-return structure is provided within said inserting tube, a spring is sleeved around the outer periphery of said inserting tube at the rear end thereof, the front end of said spring is connected to the rear end of said holding element of said interposing element, the rear end of said spring is fixed against the front end of said ring protruding stopper at the rear end of said inserting tube, and no flow-directing hole is provided on said interposing element.

28. The safety winged set according to claim 27, wherein no interposing element is provided within said inserting tube, no ring protruding stopper is provided on the interior wall of said inserting tube, and said non-return structure is directly provided on the interior wall of said inserting tube at the front end.

29. The safety winged set according to claim 28, wherein no interposing element is provided within said inserting tube, no ring protruding stopper is provided on the interior wall of said inserting tube, and said non-return structure is directly provided on the interior wall of said inserting tube at the front end.

30. The safety winged set according to claim 27, wherein said holding element is provided on the rear end or the middle outer periphery of said interposing element, and said ring protruding stopper at the front end of said holding element of said inserting tube extends to the front end of said inserting tube.

31. The safety winged set according to claim 1, wherein the outer periphery at the front end of said winged needle cover is attached thereto a bent piece, and a hemostatic cotton is connected to the upper end of said bent piece.

32. The safety winged set according to claim 31, wherein an inserting bar extends from the lower portion of said bent piece 211, and an inserting hole to be inserted by said inserting bar is provided on said winged needle cover.

33. The safety winged set according to claim 32, wherein said inserting bar at the lower portion of said bent piece is replaced by a hook, and a shaft bar to be engaged with said hook is provided on said winged needle cover 2.

34. The safety winged set according to claim 33, wherein a shaft hole is provided at the lower portion of said bent piece to be sleeved on said shaft bar.
